# EUROPEAN PATENT SPECIFICATION

(11) **EP 1 937 222 B1**
(45) Date of publication and mention of the grant of the patent: **26.02.2014**
(21) Application number: 06799087.9
(22) Date of filing: 04.10.2006
(51) Int. Cl.: A61K 9/22, A61K 9/50

(54) **NEW SUSTAINED-RELEASE MULTIPLE UNIT DOSAGE FORM**
NEUE MULTI-EINHEITS-DOSIERFORM MIT VERZÖGERTER FREISETZUNG
FORME POSOLOGIQUE UNITAIRE MULTIPLE A LIBERATION PROLONGEE

(30) Priority: 17.10.2005 KR 20050097622
(43) Date of publication of application: 02.07.2008
(73) Proprietor: GL PharmTech Corp., Gyeonggi-do 462-807 (KR)
(72) Inventor: WANG, Hun Sik, Gyeonggi-do 463-828 (KR); PARK, Jun Sang, Gyeonggi-do 463-500 (KR)
(74) Representative: Ulmann, Catherine Claire
(86) International application number: PCT/KR2006/004007
(87) International publication number: WO 2007/046590

(56) References cited:
- EP-A1- 0 421 581
- US-A- 4 828 840
- US-A1- 2004 086 566
- US-B2- 6 544 646
- DATABASE WPI Week 198909 Thomson Scientific, London, GB; AN 1989-064959 XP002687449, & JP 1 016715 A (KYOTO YAKUHIN KK) 20 January 1989 (1989-01-20)
- PASSERINI ET AL.: 'Controlled release of verapamil hydrochloride from waxy microparticles prepared by spray congealing' JOURNAL OF CONTROLLED RELEASE vol. 88, 2003, pages 263 - 275, XP004412759

## Description

### Technical Field

The present invention relates to new oral sustained-release dosage form, particularly to an oral sustained-release multiple unit dosage form capable of inhibiting the media diffusion into the core.

### Background Art

In general, a sustained-release dosage form refers to a dosage form which controls the release of drug incorporated in the dosage form in order to control the rate and extent of the absorption of the drug in the body. The dosage form is utilized to maintain the blood level of the drug as constant as possible, improve patient compliance by minimizing the number of drug administration and prevent side effects.

Differently from the single unit such as tablet, capsule, etc., the multiple unit dosage form comprises a plurality of drug-containing single units. Typical examples of the multiple unit dosage form are pellet and granule type forms. In general, the multiple unit dosage form is administered as incorporated in a single unit, e.g., tablet, capsule, etc.

The multiple unit dosage form is advantageous over the single unit dosage form in that it enables uniform drug release in oral administration. Also, although controversial, it is known that the multiple unit dosage form is known to provide more regular gastric emptying rate, or the rate of the emptying from the stomach to the small intestine, compared with the single unit dosage form. Further, it is preferred as an alternative dosage form that can reduce the side effects caused by the overly or incomplete release of drug resulting from the physical or chemical damage occurring following the oral administration of the sustained-release single unit dosage form.

The sustained-release pellet dosage form, the most widely known sustained-release multiple unit dosage form, is typically in the form of drug-containing sustained-release spherical granules or in the form of a drug-free inert pellet core coated by a drug-containing sustained-release matrix or binder and then coated again by a sustained-release matrix for controlled release.

In the latter case, or the dosage form prepared by coating the surface of an inert multiple unit cores with drug and then with a sustained-release matrix for controlled release, a water-soluble core such as sugar sphere or a water-swellable core such as cellulose sphere has been usually used as inert core (U.S. Patent No. 5783215; U.S. Patent No. 6183777; U.S. Patent No. 5633015; U.S. Patent No. 4634587; US 4828840).

Document US2004086566 discloses granules prepared by hot-melting of wax with a drug. Such prepared granules are further mixed with excipients, compressed into a tablet core. This core is further coated with controlled-release membrane. Therefore, such a formulation form is not a multiple-unit dosage form.

### Disclosure of Invention

### Technical Problem

The use of water-soluble or water-swellable core may result in the following problems.

1) The medium outside the dosage form may be diffused not only into the controlled-release membrane and the drug-containing layer but also into the core. Further, due to increased osmotic pressure in case of water-soluble core and due to increased internal mechanical pressure in case of water-swellable core, the initial release of drug increases.

2) As the medium and the drug dissolved by the medium diffuses not only outside the controlled-release membrane but also into the core, the diffusion trajectory of the drug increases, resulting in the reduction of release rate. This phenomenon becomes severer as the amount of the remaining drug decreases, thereby resulting in significant reduction of drug release rate at later stage.

3) Significant increase of osmotic pressure in case of water-soluble core and significant increase of internal mechanical pressure in case of water-swellable core may result in the rupture or damage of the controlled-release membrane, making the efficient, controlled drug release impossible.

4) The osmotic pressure inside the GI (gastrointestinal) tract may vary greatly depending on the presence or absence of food, the type of food present in the GI tract, etc. The change in osmotic pressure depending on the environmental change in the GI tract may result in the difference in the osmotic pressure inside and outside of the water-soluble or water-swellable core, thereby making it unable to maintain the originally designed drug release rate.

In order to solve these problems, U.S. Patent No. 6911217 coats a water-soluble or water-swellable core with a water-insoluble polymer layer and then with a drug-containing layer or a controlled-release membrane. In spite of superior effect, this technique is disadvantageous in that the process of introducing an additional water-insoluble layer is complicated and that the layer has to be sufficiently thick in order to prevent the diffusion of drug into the core. Further, even if it is possible to prevent the diffusion of drug into the core, the diffusion of the medium into the core cannot be inhibited fundamentally.

Through consistent researches, the present inventors found out that the aforementioned problems can be solved completely when waxes, higher fatty acids or glyceryl fatty acids are used as multiple unit cores and also that the inflow of the medium into the core can be inhibited because of the hydrophobicity of the core. Thus, an object of the present invention is to provide a sustained-release multiple unit dosage form that is capable of inhibiting the penetration of the medium into the multiple unit cores and enables more efficient controlled release of drug.

### Technical Solution

To attain the aforesaid object, the present invention provides a sustained-release multiple unit dosage form comprising a multiple unit cores and a drug-containing layer and a controlled-release membrane formed on the surface of the core.

The multiple unit cores may be made of at least one selected from the group consisting of waxes, higher fatty acids and glyceryl fatty acids. Since the multiple unit cores is hydrophobic with little affinity to water, the medium outside of the core cannot diffuse into the core. Consequently, the aforementioned problems - increased initial release caused by the diffusion of the medium into the core, reduced release at later stage and damage of the controlled-release membrane - can be avoided and, especially, the inhibition of the diffusion of the medium into the core may improve the productivity by greatly reducing the coated amount or coating rate of the controlled-release membrane as compared with the conventional water-swellable or water-soluble core. In addition, the use of water-insoluble, hydrophobic core restricts the region that can be penetrated by the medium neighboring the dosage form to a much smaller area compared with the drug-containing layer, the controlled-release layer, etc. of the core, it becomes possible to avoid the unwanted change of drug release rate which results the physical property difference of the core itself, for example, increased osmotic pressure caused by the dissolution of the water-soluble core itself, increased internal mechanical pressure in the water-swellable core due to the water-wicking, etc.

The morphology of the multiple unit cores is not particularly restricted, but a spherical form is preferable and a size of 0.1 mm to 2 mm is preferable.

The multiple unit cores may be made of a wax such as microcrystalline wax, beeswax (white beeswax), carnauba wax, sperm wax, emulsifying wax, paraffin, yellow beeswax, cetyl ester wax, etc. or a higher fatty acid such as lauric acid, myristic acid, palmitic acid, stearic acid, arachidic acid, etc. Also, a glyceryl fatty acid such as hydrogenated castor oil, hydrogenated vegetable oil type I, glyceryl behenate, glyceryl palmitostearate, glyceryl stearate, etc. may be used.

In general, a coating machine is used to coat a drug-containing layer and/or a controlled-release membrane on the surface of the multiple unit cores. Since the coating process is frequently performed while applying hot air to facilitate drying, the multiple unit cores preferably has a melting point of at least 40°C, more preferably at least 60°C. But, this is not compulsory because the coating process can also be performed using air at room temperature.

The present invention is characterized in that the drug is coated on the surface of the multiple unit cores. Depending on situations, the use of a binder may be unnecessary. But, usually, a binder is used to coat the drug on the multiple unit cores. The binder may be a water-soluble, water-swellable or water-insoluble polymer well known by those skilled in the art or any material other than polymer that can bind the drug onto the multiple unit cores. The drug-containing layer may comprise, in addition to the binder, pharmaceutically allowed additives such as excipient, disintegrant, colorant, opacifier, lubricant, plasticizer, etc. In addition to binding the drug onto the core, the binder itself may function as the controlled-release membrane.

The present invention is also characterized in that the drug-containing layer is coated with the controlled-release membrane.

The controlled-release membrane is used to prevent the drug included in the drug-containing layer from being released in short time and control the drug release rate and may comprise a single or a plurality of membranes.

The controlled-release membrane may be made of a water-insoluble polymer, for example, ethylcellulose (Aquacoat(r), FMC; Surelease, ColorCon), polyvinylacetate (Kollicoat(r) SR, BASF), ammoniomethacrylates (Eudragit(r) RS or RL, Degussa), poly(ethyl acrylate) methyl methacrylate (Eudragit(r) NE30D, Degussa), cellulose acetate, etc.

For further control of release rate, the controlled-release membrane may selectively comprise, in addition to the water-insoluble polymer, a water-soluble polymer, for example, hydroxypropylmethylcellulose, hydroxypropylcellulose, methylcellulose, polyvinylalcohol, polyvinylpyrolidone, polyethyleneglycol, etc.

The controlled-release membrane may further comprise pharmaceutically allowed additive, for example, excipient, disintegrant, colorant, opacifier, lubricant, plasticizer, etc.

### Advantageous Effects

Through effective controlled release of drug, the present invention can reduce side effects and provide long-lasting medicinal effect. Further, it provides better productivity compared with the water-swellable or water-soluble multiple unit cores.

### Brief Description of the Drawings

Fig. 1 shows the time-dependent drug release profile for Example 1 and Comparative Example 1.
Fig. 2 shows the dissolution rate for Example 1 and Comparative Example 1 per unit time.

### Best Mode for Carrying Out the Invention

Hereinafter, the embodiments of the present invention will be described in detail referring to examples.

### <Example 1>

About 563 g of carnauba wax (20-25 mesh) was put in a Wurster-type coater (GPCG1, Glatt) as multiple unit cores and then a drug-containing layer was coated. The drug-containing layer coating solution had been prepared by dissolving 28 g of tolterodine L-tartrate, a pharmacologically active ingredient, and 7 g of hydroxypropylmethylcellulose, a binder, in 798 g of purified water and dispersing 1.4 g of talc as lubricant.

After the coating of the drug-containing layer had been completed, a controlled-release membrane was coated using the same coating machine. The controlled-release membrane coating solution had been prepared by dissolving 15.4 g of hydroxypropylmethylcellulose, a water-soluble polymer, in 371 g of purified water, adding 370.7 g of Surelease(r) and then dispersing talc. The coated multiple unit cores was kept in a tray oven dryer (40°C) for 24 hours.

### <Example 2>

About 563 g of stearic acid (18-20 mesh) was put in a Wurster-type coater (GPCG1, Glatt) as multiple unit cores and then a drug-containing layer was coated. The drug-containing layer coating solution had been prepared by dissolving 28 g of tolterodine L-tartrate, a pharmacologically active ingredient, and 28 g of hydroxypropylmethylcellulose, a binder, in 798 g of purified water and dispersing 5.6 g of talc as lubricant.

After the coating of the drug-containing layer had been completed, a controlled-release membrane was coated using the same coating machine. The controlled-release membrane coating solution had been prepared by dissolving 23.2 g of hydroxypropylmethylcellulose, a water-soluble polymer, in 556 g of purified water, adding 556 g of Surelease(r) and then dispersing talc. The coated multiple unit cores was kept in a tray oven dryer (50°C) for 48 hours.

### [41] <Example 3>

About 563 g of carnauba wax (18-20 mesh) was put in a Wurster-type coater (GPCG1, Glatt) as multiple unit cores and then a drug-containing layer was coated. The drug-containing layer coating solution had been prepared by dissolving 70 g of ibudilast, a pharmacologically active ingredient, in 350 g of ethanol (KP).

After the coating of the drug-containing layer had been completed, a controlled-release membrane was coated using the same coating machine. The controlled-release membrane coating solution had been prepared by dissolving 12.3 g of hydroxypropylmethylcellulose, a water-soluble polymer, in 602 g of ethanol (KP) and adding 40.8 g of ethylcellulose, 8.2 g of dibutyl sebacate and 53.1 g of magnesium stearate.

### <Comparative Example>

960 g of water-soluble sugar sphere (20-25 mesh; IPS) was put in a Wurster-type coater (GPCG1, Glatt) as multiple unit cores and then a drug-containing layer was coated. The drug-containing layer coating solution had been prepared by dissolving 28 g of tolterodine L-tartrate and 28 g of hydroxypropylmethylcellulose, a binder, in 798 g of purified water and dispersing 5.6 g of talc as lubricant.

After the coating of the drug-containing layer had been completed, a controlled-release membrane was coated using the same coating machine. The controlled-release membrane coating solution had been prepared by dissolving 23.2 g of hydroxypropylmethylcellulose, a water-soluble polymer, in 556 g of purified water, adding 556 g of Surelease(r) and then dispersing 32.4 g of talc. The coated multiple unit cores was kept in a tray oven dryer (40 °C) for 24 hours.

### <Dissolution Test>

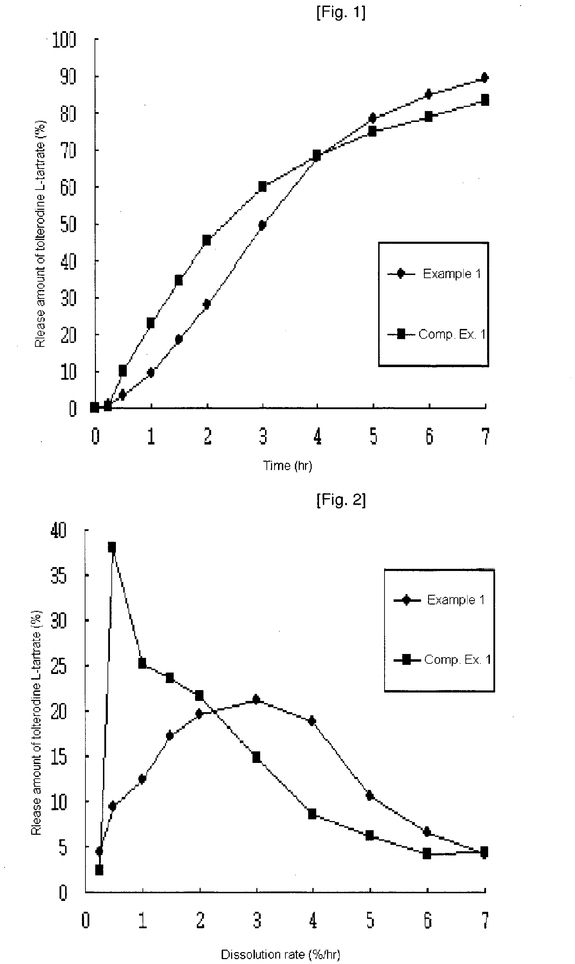

Dissolution test was performed for the coated multiple unit coress prepared in Example 1 and Comparative Example 1, in accordance with the basket method (100 rpm) of Korea Pharmacopeia using a pH 6.8 phosphate buffer (900 mL). The drug release profile and dissolution rate are shown in Figs. 1 and 2.

Both examples showed similar total drug release time of about 7 hours. However, Example 1 showed much less initial release rate and more consistent release rate at later stage compared with Comparative Example 1. As seen in Fig. 2, Comparative Example 1, in which the water-soluble multiple unit cores was used, showed extraordinarily high initial dissolution rate (initial burst), which decreased rapidly. In contrast, Example 1, in which the hydrophobic wax core was used, showed adequately controlled initial dissolution rate and relatively constant dissolution rate after 2 hours. Thus, it can be seen that the present invention provides more effective control of drug release.

In addition, although the total drug release time is almost the same, the coated amount of the controlled-release membrane in Example 1 was just about 67 % that of Comparative Example 1. This implies that the present invention can significantly improve productivity.

### Industrial Applicability

By inhibiting the media diffusion outside the dosage form into the core, the oral sustained-release dosage form of the present invention can reduce side effects and provide long-lasting medicinal effect. Further, it provides better productivity compared with the water-swellable or water-soluble multiple unit cores.

## Claims

1. A sustained-release multiple unit dosage form comprising:
1) hydrophobic multiple unit cores comprising at least one selected from the group consisting of a wax, a higher fatty acid and a glyceryl fatty acid;
2) a drug-containing layer coated on the surface of said core; and
3) a controlled-release membrane coated on the surface of said layer.

2. The sustained-release multiple unit dosage form as set forth in Claim 1, wherein the wax, higher fatty acid or glyceryl fatty acid has a melting point of at least 40°C.

3. The sustained-release multiple unit dosage form as set forth in Claim 1, wherein the hydrophobic multiple unit cores is spherical.

4. The sustained-release multiple unit dosage form as set forth in Claim 1, wherein the hydrophobic multiple unit cores has a size ranging from 0.1 mm to 2 mm.

5. The sustained-release multiple unit dosage form as set forth in Claim 1, wherein the controlled-release membrane comprises a water-insoluble polymer.

6. The sustained-release multiple unit dosage form as set forth in Claim 1, wherein the controlled-release membrane comprises a water-insoluble polymer and, optionally, a water-soluble polymer.

7. The sustained-release multiple unit dosage form as set forth in Claim 1, which comprises at least one controlled-release membrane.

8. The sustained-release multiple unit dosage form as set forth in Claim 1, wherein the hydrophobic multiple unit cores comprises at least one selected from the group consisting of microcrystalline wax, beeswax, carnauba wax, sperm wax, emulsifying wax, paraffin, yellow beeswax, cetyl ester wax, lauric acid, myristic acid, palmitic acid, stearic acid, arachidic acid, hydrogenated castor oil, hydrogenated vegetable oil type I, glyceryl behenate, glyceryl palmitostearate and glyceryl stearate.

9. The sustained-release multiple unit dosage form as set forth in Claim 1, wherein the hydrophobic multiple unit cores comprises carnauba wax.

10. The sustained-release multiple unit dosage form as set forth in Claim 5 or Claim 6, wherein the water-insoluble polymer is at least one selected from the group consisting of ethylcellulose, polyvinylacetate, ammoniomethacrylate, poly(ethyl acrylate) methyl methacrylate and cellulose acetate.

11. The sustained-release multiple unit dosage form as set forth in Claim 5 or Claim 6, wherein the water-insoluble polymer is ethylcellulose.

12. The sustained-release multiple unit dosage form as set forth in Claim 6, wherein the water-soluble polymer is hydroxypropylmethylcellulose.

## Patentansprüche

1. Multieinheits-Dosierform mit verzögerter Freisetzung, umfassend:
1) hydrophobe Mehreinheitenkerne, die wenigstens eines ausgewählt aus der Gruppe bestehend aus einem Wachs, einer höheren Fettsäure und einer Glycerolfettsäure um-fassen;
2) eine arzneistoffhaltige Schicht, mit der die Oberfläche des Kerns beschichtet ist; und
3) eine Membran für gesteuerte Freisetzung, mit der die Oberfläche der Schicht beschichtet ist.

2. Multieinheits-Dosierform mit verzögerter Freisetzung nach Anspruch 1, wobei das Wachs, die höhere Fettsäure oder die Glycerolfettsäure einen Schmelzpunkt von wenigstens 40°C aufweist.

3. Multieinheits-Dosierform mit verzögerter Freisetzung nach Anspruch 1, wobei die hydrophoben Mehreinheitenkerne kugelförmig sind.

4. Multieinheits-Dosierform mit verzögerter Freisetzung nach Anspruch 1, wobei die hydrophoben Mehreinheitenkeme eine Größe im Bereich von 0,1 mm bis 2 mm aufweisen.

5. Multieinheits-Dosierform mit verzögerter Freisetzung nach Anspruch 1, wobei die Membran für gesteuerte Freisetzung ein wasserunlösliches Polymer umfasst.

6. Multieinheits-Dosierform mit verzögerter Freisetzung nach Anspruch 1, wobei die Membran für gesteuerte Freisetzung ein wasserunlösliches Polymer und, optional, ein wasserlösliches Polymer umfasst.

7. Multieinheits-Dosierform mit verzögerter Freisetzung nach Anspruch 1, die wenigstens eine Membran für gesteuerte Freisetzung umfasst.

8. Multieinheits-Dosierform mit verzögerter Freisetzung nach Anspruch 1, wobei die hydrophoben Mehreinheitenkeme wenigstens eines ausgewählt aus der Gruppe bestehend aus mikrokristallinem Wachs, Bienenwachs, Carnaubawachs, Walrat, Emulgierwachs, Paraffin, gelbem Bienenwachs, Cetylesterwachs, Laurinsäure, Myristinsäure, Palmitinsäure, Stearinsäure, Arachinsäure, gehärtetem Rizinusöl, gehärtetem pflanzlichem Öl vom Typ I, Glycerolbehenat, Glycerolpalmitatstearat und Glycerolstearat umfassen.

9. Multieinheits-Dosierform mit verzögerter Freisetzung nach Anspruch 1, wobei die hydrophoben Mehreinheitenkerne Carnaubawachs umfassen.

10. Multieinheits-Dosierform mit verzögerter Freisetzung nach Anspruch 5 oder Anspruch 6, wobei das wasserunlösliche Polymer wenigstens eines ausgewählt aus der Gruppe bestehend aus Ethylcellulose, Polyvinylacetat, Ammoniummethacrylat, Poly-(ethylacrylat)methylmethacrylat und Celluloseacetat ist.

11. Multieinheits-Dosierform mit verzögerter Freisetzung nach Anspruch 5 oder Anspruch 6, wobei das wasserunlösliche Polymer Ethylcellulose ist.

12. Multieinheits-Dosierform mit verzögerter Freisetzung nach Anspruch 6, wobei das wasserlösliche Polymer Hydroxypropylmethylcellulose ist.

## Revendications

1. Forme posologique à unités multiples à libération prolongée comprenant :
1) des coeurs unitaires multiples hydrophobes comprenant au moins l'un du groupe constitué par une cire, un acide gras supérieur et un acide gras glycérylique ;
2) une couche contenant un médicament déposée sous forme de revêtement sur la surface dudit coeur ; et
3) une membrane de libération prolongée déposée sous forme de revêtement sur la surface de ladite couche.

2. Forme posologique à unités multiples à libération prolongée selon la revendication 1, dans laquelle la cire, l'acide gras supérieur ou l'acide gras glycérylique a un point de fusion d'au moins 40°C.

3. Forme posologique à unités multiples à libération prolongée selon la revendication 1, dans laquelle les coeurs unitaires multiples hydrophobes sont sphériques.

4. Forme posologique à unités multiples à libération prolongée selon la revendication 1, dans laquelle les coeurs unitaires multiples hydrophobes ont une taille située dans la plage allant de 0,1 mm à 2 mm.

5. Forme posologique à unités multiples à libération prolongée selon la revendication 1, dans laquelle la membrane à libération prolongée comprend un polymère insoluble dans l'eau.

6. Forme posologique à unités multiples à libération prolongée selon la revendication 1, dans laquelle la membrane à libération prolongée comprend un polymère insoluble dans l'eau et éventuellement un polymère soluble dans l'eau.

7. Forme posologique à unités multiples à libération prolongée selon la revendication 1, qui comprend au moins une membrane de libération prolongée.

8. Forme posologique à unités multiples à libération prolongée selon la revendication 1, dans laquelle les coeurs unitaires multiples hydrophobes comprennent au moins l'un du groupe constitué par la cire microcristalline, la cire d'abeilles, la cire de carnauba, le spermacéti, une cire émulsionnante, la paraffine, la cire d'abeilles jaune, une cire d'ester cétylique, l'acide laurique, l'acide myristique, l'acide palmitique, l'acide stéarique, l'acide arachidique, l'huile de ricin hydrogénée, une huile végétale hydrogénée de type I, le béhénate de glycéryle, le palmitostéarate de glycéryle et le stéarate de glycéryle.

9. Forme posologique à unités multiples à libération prolongée selon la revendication 1, dans laquelle les coeurs unitaires multiples hydrophobes comprennent de la cire de carnauba.

10. Forme posologique à unités multiples à libération prolongée selon la revendication 5 ou la revendication 6, dans laquelle le polymère insoluble dans l'eau est au moins l'un du groupe constitué par l'éthylcellulose, le poly(acétate de vinyle), le méthacrylate d'ammonium, le méthacrylate de méthyle poly(acrylate d'éthyle) et l'acétate de cellulose.

11. Forme posologique à unités multiples à libération prolongée selon la revendication 5 ou la revendication 6, dans laquelle le polymère insoluble dans l'eau est l'éthylcellulose.

12. Forme posologique à unités multiples à libération prolongée selon la revendication 6, dans laquelle le polymère soluble dans l'eau est l'hydroxypropylméthylcellulose.
